# EUROPEAN PATENT APPLICATION

(11) **EP 4 811 316 A1**
(43) Date of publication of application: **23.09.2026**
(21) Application number: 25218114.4
(22) Date of filing: 24.11.2025
(51) Int. Cl.: G06V 40/13, A61B 5/0537, A61B 5/1174, A61B 5/279, G06V 40/12, G06V 40/16, G06V 40/50

(54) **BODY FAT SCALE AND CONTROL METHOD THEREOF**

(30) Priority: 19.03.2025 CN 202510325883
(71) Applicant: Shenzhen Unique Scales Co., Ltd., Shenzhen City, Guangdong 518117 (CN)
(72) Inventor: YU, Huayun, Shenzhen City, 518117 (CN); YANG, Xianwen, Shenzhen City, 518117 (CN)
(74) Representative: Zacco Sweden AB

(57) **Abstract**

This application provides a body fat scale and a control method thereof. The body fat scale includes a biometric recognizer and a controller. The biometric recognizer includes a first fingerprint recognizer and a second fingerprint recognizer. The first fingerprint recognizer includes a first fingerprint recognition panel adjustable by an angle; and the second fingerprint recognizer includes a second fingerprint recognition panel that forms the first inclination angle with a vertical plane. An angle of each component conforms to a natural angle at which a finger of the user comes into contact with the body fat scale, which improves user experience. The controller is configured to: determine a target fingerprint recognizer suitable for the user; in response to a fingerprint enrollment operation of the user via the target fingerprint recognizer, determine a first identifier corresponding to a first fingerprint image; based on the first identifier, establish a matching relationship between the first initial data and basic data corresponding to the first identifier; and learn use habits and the like of the users during use processes of the users to adapt to operational characteristics of different users and quickly and accurately determine identities of the users.

## Description

### TECHNICAL FIELD

This application pertains to the field of electronic measurement, and in particular, relates to a body fat scale and a control method thereof.

### BACKGROUND

As living standards of people are improved, people are paying increasing attention to health and body management. In daily life, users manage their health and fitness via body fat scales, which help them learn their body indicators, for example, body weight, body fat percentage, BMI, visceral fat, and muscle mass.

In the related art, the body fat scales establish connections with terminal devices such as smartphones and tablet computers through communication means like Bluetooth and Wi-Fi, thereby achieving collaboration with applications on these terminal devices. During such process, the user needs to input basic data such as height, gender, and age into the application; and the body fat scale is equipped with a pressure sensor and an electrode plate for measuring a pressure value, an impedance value, and other measurement data generated when the user stands on the scale body, and the user needs to manually select basic data of the user on an operation interface of the body fat scale or through a supporting application, to match and associate basic data of the user with the measurement data, thereby subsequently performing data processing and determining indicator data.

However, in conventional use scenarios, when the body fat scale is used by multiple users, matching the basic data via the foregoing manual selection often leads to inaccurate identification of the users, and as a result, indicator data and analyses are further inaccurate, thereby causing poor user experience.

### SUMMARY

This application provides a body fat scale and a control method thereof, so that an identity of a user during a use process of the user can be quickly and accurately determined.

According to the first aspect, a body fat scale is provided, including a biometric recognizer, a measurement device, a controller, and a communication module, where the controller is communicatively connected to the biometric recognizer, the measurement device, and the communication module. The biometric recognizer includes a first fingerprint recognizer and a second fingerprint recognizer, where the first fingerprint recognizer is disposed on the first surface of the body fat scale, and the first fingerprint recognizer includes a first fingerprint recognition panel adjustable by an angle and a first sensor; and the second fingerprint recognizer is disposed on a side surface of the body fat scale, the second fingerprint recognizer includes a second fingerprint recognition panel and a second sensor, and there is a first inclination angle between the second fingerprint recognition panel and a vertical plane. The measurement device includes an electrode and a pressure sensor.

The controller is configured to: when the number of uses of the body fat scale reaches a preset number and it is detected that a user is in a stable state on the body fat scale, obtain the first initial data of the user, where the first initial data includes bioimpedance data and pressure distribution data; input the first initial data, historical use data and user preference analysis data into a preset recognition model to determine a target fingerprint recognizer suitable for the user; in response to a fingerprint enrollment operation of the user via the target fingerprint recognizer, obtain the first fingerprint image of the user and determine the first identifier corresponding to the first fingerprint image, where the target fingerprint recognizer is the first fingerprint recognizer or the second fingerprint recognizer; and based on the first identifier, establish a matching relationship between the first initial data and basic data corresponding to the first identifier, and determine indicator data of the user.

The body fat scale is equipped with multiple fingerprint recognizers and features a layout designed with full consideration for ergonomics and usage scenarios. Some fingerprint recognizers are disposed on the surface of the body fat scale, while other fingerprint recognizers are hidden on the side surface of the body fat scale, thereby providing more options for users with different use habits. In addition, during the use of the body fat scale, the body fat scale continuously learns the use habit, user preference analysis data, and the like of the user to adapt to operating characteristics of different users, and then guides the user to enroll a fingerprint through the target fingerprint recognizer among the multiple fingerprint recognizers, thereby quickly and accurately determining the identity of the user.

In a possible implementation, the first fingerprint recognizer further includes a universal connection mechanism, the universal connection mechanism is configured to movably connect the first fingerprint recognition panel to the body fat scale, the first fingerprint recognition panel is provided with the first sensor, and the second fingerprint recognition panel is provided with the second sensor.

The universal connection mechanism is disposed in the first fingerprint recognizer, to configure the first fingerprint recognition panel as a panel adjustable by an angle. The users can manually rotate the first fingerprint recognition panel in the first fingerprint recognizer to a specific angle, so that a user with different hand gestures or different users enroll fingerprint images through the first fingerprint recognition panel .

In a possible implementation, the universal connection mechanism further includes an angle sensor and a motor, and after determining the target fingerprint recognizer suitable for the user, the controller is further configured to: if the target fingerprint recognizer is the first fingerprint recognizer, analyze the historical use data to determine a target angle of the first fingerprint recognition panel, where the historical use data includes use frequency of the first fingerprint recognizer and an initial angle that is obtained via the angle sensor when the user enrolls a fingerprint and that corresponds to the first fingerprint recognition panel; and control the motor to adjust the angle of the first fingerprint recognition panel to the target angle.

It should be understood that the universal connection mechanism is driven via a motor to drive the fingerprint recognition panel to rotate, thereby implementing automatic angle adjustment. With this structure, multi-angle adjustment within a relatively wide range can be implemented, thereby meeting needs of different users (with, for example, different heights and use habits) to enroll fingerprints in comfortable postures. In addition, during use, before the user enrolls the fingerprint, the fingerprint recognition panel in the corresponding fingerprint recognizer can be adjusted to a suitable angle.

In a possible implementation, after obtaining the first fingerprint image of the user in response to the fingerprint enrollment operation of the user via the target fingerprint recognizer, the controller is further configured to: identify and analyze the first fingerprint image based on user information stored in the body fat scale; if the first fingerprint image is not recognized, perform a matching degree analysis respectively on a characteristic of the first fingerprint image and characteristics of fingerprints that have been collected by the first fingerprint recognizer and the second fingerprint recognizer, to obtain a corresponding similarity; and use the fingerprint recognizer with the highest similarity as an updated target fingerprint recognizer.

After the first fingerprint image is obtained, it is possible that the first fingerprint image is unrecognized during the process of identifying and analyzing the first fingerprint image. To improve a fault tolerance rate of the solution and user experience, if the first fingerprint image is unrecognized, the user is instructed to enroll the fingerprint via another fingerprint recognizer based on the analysis.

In a possible implementation, when the number of uses of the body fat scale does not reach a preset number and it is detected that the user is in a stable state on the body fat scale, the controller is further configured to: obtain the second fingerprint image and the second initial data of the user, where the second fingerprint image is an image collected via the first fingerprint recognizer and/or the second fingerprint recognizer; analyze the second fingerprint image and the second initial data to obtain the historical use data and user preference analysis data of the user; and optimize the preset recognition model based on the second fingerprint image, the second initial data, the historical use data, and the user preference analysis data, to obtain an optimized preset recognition model.

In daily use scenarios of the body fat scale, intelligence of the body fat scale is gradually improved as the number of times of using the body fat scale by the user increases. Each time the user completes a measurement, the body fat scale collects the second fingerprint image to accurately determine the identity of the user. The body fat scale uses the second fingerprint image, the second initial data, historical use data, and user preference analysis data together as input data to perform a comprehensive optimization of the preset recognition model. During continuous iterative operations, the preset recognition model gradually adjusts its internal parameters to improve recognition accuracy of an individual characteristic and a behavioral habit of the user. In this way, the optimized preset recognition model is obtained, to further provide a more accurate target fingerprint recognition model for the user in the subsequent use process and deliver a customized service to the user.

In a possible implementation, the first fingerprint recognizer further includes a first indicator light, and the second fingerprint recognizer further includes a second indicator light; and after determining the target fingerprint recognizer suitable for the user, the controller is further configured to: generate prompt information, where the prompt information is used to instruct the user to enroll a fingerprint via the target fingerprint recognizer. When the prompt information indicates that the first fingerprint recognizer is the target fingerprint recognizer, the first indicator light is driven to emit light based on a preset mode; or when the prompt information indicates that the second fingerprint recognizer is the target fingerprint recognizer, the second indicator light is driven to emit light based on the preset mode, where the preset mode includes a constant-on mode or a flashing mode.

The user is instructed, via the generated prompt information, to enroll the fingerprint via the target fingerprint recognizer. The prompt information can be presented in different forms to achieve a purpose of prompting the user.

In a possible implementation, the first fingerprint recognizer further includes the first LED light source, and the first sensor includes an optical fingerprint sensor and a capacitive fingerprint sensor; and the controller is further configured to: obtain heart rate data of the user, where the heart rate data is used to obtain the historical use data of the user to optimize the preset recognition model, and to be input into the preset recognition model for determining the target fingerprint recognizer suitable for the user.

Because more input data can cover more features and changes, the preset recognition model can learn more complex and subtle patterns and rules, which reduces a risk of overfitting and enhances a generalization capability on unknown data, thereby improving accuracy of the preset recognition model. Therefore, on the premise that the fingerprint recognition function is not affected, a dedicated heart rate signal processing algorithm can be added to the controller, to subject a collected photoplethysmography signal to processing such as filtering, amplification, and feature extraction, thereby calculating the heart rate data of the user. The heart rate data is used as the data for training, optimizing, and using the preset model.

In a possible implementation, the body fat scale includes a handle and a scale body; and the biometric recognizer further includes a facial recognizer, the first fingerprint recognizer and the facial recognizer are disposed on the second surface of the handle, and the second fingerprint recognizer is disposed on the side surface of the scale body.

The body fat scale that can be divided into the handle and the scale body is equipped with multiple fingerprint recognizers. Some fingerprint recognizers are disposed on the surface of the handle, while other fingerprint recognizers are hidden on the side surface of the scale body, thereby providing more options for users with different use habits. With this design, regardless of the posture of the user when stepping onto the body fat scale, and regardless of a height, a hand shape or a finger characteristic of the user, the user can conveniently and comfortably put the finger on one of the fingerprint recognizers, which greatly improves convenience of obtaining the fingerprint image and optimizes the user experience.

In a possible implementation, the first sensor includes an optical fingerprint sensor and a capacitive fingerprint sensor, and the second sensor includes an optical fingerprint sensor and a capacitive fingerprint sensor.

The first sensor and the second sensor serve as integrated sensors, which combine the optical fingerprint sensor and the capacitive fingerprint sensor. The integration of multiple types of sensors not only increases the recognition speed but also enhances its accuracy. Even when the finger has slight stains or is damp, the fingerprint image can still be quickly and accurately obtained.

In a possible implementation, the biometric recognizer further includes a toe print recognizer, the toe print recognizer is disposed on the first surface of the body fat scale, and the user performs a toe print enrollment operation via the toe print recognizer, to obtain a toe print image of the user and determine the first identifier corresponding to the toe print image.

In a possible implementation, the toe print recognizer includes the third fingerprint recognition panel adjustable by an angle and the third sensor, the third fingerprint recognition panel is movably connected to the body fat scale via a universal connection mechanism, and the third fingerprint recognition panel is provided with the third sensor.

In a possible implementation, the third sensor includes an optical fingerprint sensor and a capacitive fingerprint sensor.

According to a second aspect, a body fat scale controlling method is provided, applied to the body fat scale according to any one of implementations of the first aspect, where the controlling method includes: when the number of uses of the body fat scale reaches a preset number and it is detected that a user is in a stable state on the body fat scale, obtaining the first initial data of the user, where the first initial data includes bioimpedance data and pressure distribution data; inputting the first initial data, historical use data and user preference analysis data into a preset recognition model to determine a target fingerprint recognizer suitable for the user; in response to a fingerprint enrollment operation of the user via the target fingerprint recognizer, obtaining the first fingerprint image of the user and determining the first identifier corresponding to the first fingerprint image, where the target fingerprint recognizer is the first fingerprint recognizer or the second fingerprint recognizer; and based on the first identifier, establishing a matching relationship between the first initial data and basic data corresponding to the first identifier, and determining indicator data of the user.

According to a third aspect, a control apparatus is provided, including a unit for performing any one of the body fat scale controlling methods in the second aspect. The control apparatus may be the body fat scale or a chip inside the body fat scale.

According to the fourth aspect, a computer-readable storage medium is provided, where the computer-readable storage medium stores a computer program, where the computer program, when executed by the control apparatus, causes the control apparatus to perform the control method for the body fat scale according to any one of the second aspect.

According to the fifth aspect, a computer program product is provided, where the computer program product includes a computer program, where the computer program, when executed by the control apparatus, causes the control apparatus to perform the control method for the body fat scale according to any one of the second aspect.

It can be understood that, for beneficial effects of the foregoing second to fifth aspects, refer to detailed description in the foregoing first aspect. Details are not described herein again.

### BRIEF DESCRIPTION OF THE DRAWINGS

FIG. 1 is a schematic diagram of a body fat scale and communication between the body fat scale and a terminal device;
FIG. 2 is a schematic diagram of a system architecture of a body fat scale provided in an embodiment of this application;
FIG. 3 is a schematic structural diagram of a body fat scale provided in an embodiment of this application;
FIG. 4 is a schematic structural diagram of another body fat scale provided in an embodiment of this application;
FIG. 5 is a schematic diagram of the body fat scale in FIG. 4 in another form;
FIG. 6 is a schematic diagram of fingerprint enrollment via the first fingerprint recognizer in a body fat scale provided in an embodiment of this application;
FIG. 7 is a schematic diagram of fingerprint enrollment via the first fingerprint recognizer in a body fat scale provided in an embodiment of this application;
FIG. 8 is a schematic diagram of the first fingerprint recognizer in a body fat scale provided in an embodiment of this application;
FIG. 9 is a schematic structural diagram of still another body fat scale provided in an embodiment of this application;
FIG. 10 is a schematic flowchart of a body fat scale controlling method provided in an embodiment of this application;
FIG. 11 is a schematic flowchart illustrating an analysis process in a body fat scale controlling method provided in an embodiment of this application;
FIG. 12 is a schematic diagram of a body fat scale controlling apparatus provided in an embodiment of this application;
FIG. 13 is a schematic structural diagram of a body fat scale provided in this application;
FIG. 14 is a schematic structural diagram of the third body fat scale provided in an embodiment of this application; and
FIG. 15 is a schematic structural diagram of the fourth body fat scale provided in an embodiment of this application.

### DETAILED DESCRIPTION OF EMBODIMENTS

The following describes technical solutions in the embodiments of this application with reference to accompanying drawings in the embodiments of this application. In description of the embodiments of this application, unless otherwise stated, "/" means "or", for example, A/B can mean A or B. "and/or" herein is only an association relationship for describing associated objects and indicates that three relationships may exist. For example, A and/or B may mean the following three cases: Only A exists, both A and B exist, and only B exists. The terms such as "first", "second", and "third" are merely intended for a purpose of description, and shall not be understood as an indication or implication of relative importance or implicit indication of a quantity of indicated technical features. Therefore, a feature with a determiner such as "first", "second", and "third" can expressly or implicitly include one or more features.

For the purpose of illustration rather than limitation, the following describes specific details such as a specific system structure and technology, to facilitate a thorough understanding of the embodiments of this application. However, a person skilled in the art should understand that this application can also be implemented in other embodiments without these details. In other cases, detailed descriptions of well-known systems, apparatuses, circuits, and methods are omitted, to prevent unnecessary details from causing distraction from the description of this application.

As living standards of people are improved, people are paying increasing attention to health and body management. In daily life, users manage their health and fitness via body fat scales, which help them learn their body indicators, for example, body weight, body fat percentage, visceral fat, and muscle mass.

The body fat scale is usually equipped with a pressure sensor and an electrode plate and used to measure a variety of data. When a user steps onto the body fat scale, body weight of the user is evenly applied to a surface of the scale body. The pressure sensor deforms under the action of pressure, causing a change in resistance or capacitance value. This electrical change is converted into an electrical signal, and the electrical signal is subjected to processing such as amplification and filtering, and finally weight data of the user is presented in a digital form. In addition, when feet of the user or feet and hands of the user come into contact with the electrode plate of the body fat scale, the electrode plate applies a weak and safe electric current to the body of the user. This current circulates through tissues and body fluids of the user in vivo. Since a fat tissue, a muscle tissue, and the like have different impedance to the current, the electrode plate can detect a change in impedance generated when the current is transmitted through different tissues of the user, thereby obtaining the impedance value.

In the related art, the body fat scales establish connections with terminal devices such as smartphones and tablet computers through communication means like Bluetooth and Wi-Fi, thereby achieving collaboration with applications on these terminal devices. The application on the terminal device is used to collect basic data of the user, for example height, gender, and age, and transmit the basic data to the body fat scale for storage.

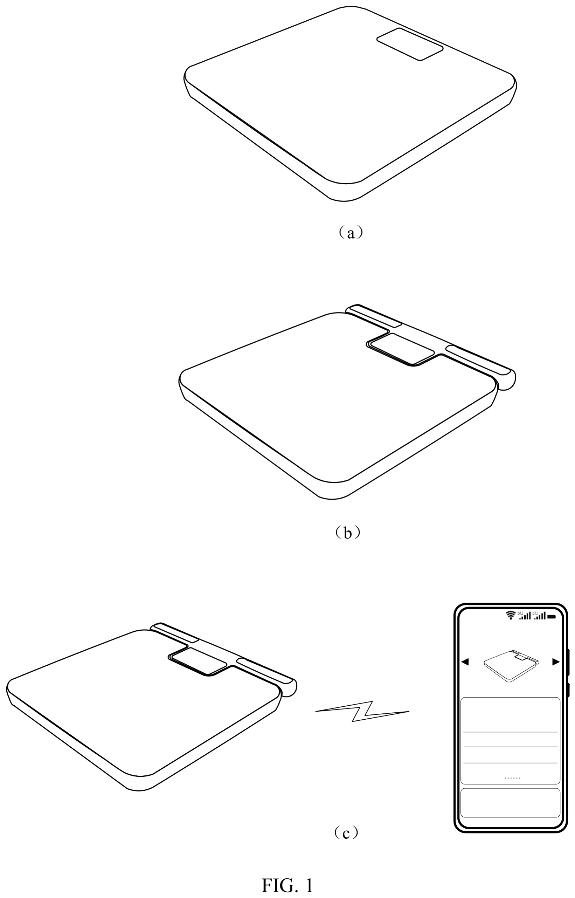

The following describes the body fat scale in the related art with reference to the accompanying drawing. FIG. 1 is a schematic diagram of a body fat scale and communication between the body fat scale and a terminal device. As shown in figure a in FIG. 1, the body fat scale can be a four-electrode body fat scale, and the four-electrode body fat scale includes a measurement device, a display, a communication module, and a power supply. Electrodes in the measurement device include four electrode plates that are disposed on a surface of a scale body of the four-electrode body fat scale. As shown in figure b in FIG. 1, the body fat scale can also be an eight-electrode body fat scale, and the eight-electrode body fat scale includes a measurement device, a display, a communication module, and a power supply. Electrodes in the measurement device include eight electrode plates, four electrode plates are disposed on a surface of a scale body of the eight-electrode body fat scale, and the other four electrode plates are disposed on a surface of a handle of the eight-electrode body fat scale. It should also be understood that there is no limitation on the number of electrode plates of the body fat scale.

It should be understood that the electrode plates can be external electrode plates or internal electrode plates. As shown in figure b in FIG. 1, the electrode plates on the handle are external electrode plates. As shown in figures a and b in FIG. 1, the electrode plates on the scale body are internal electrode plates.

As shown in figure c of FIG. 1, taking the eight-electrode body fat scale as an example, the eight-electrode body fat scale establishes a communication connection with a mobile phone through wireless communication methods such as Bluetooth and Wi-Fi. When the eight-electrode body fat scale is used for the first time, a communication connection is established between the mobile phone and the eight-electrode body fat scale through application A on the mobile phone, and basic data of user X, basic data of user Y, and the like are input into application A. When being used again, the eight-electrode body fat scale receives basic data of each user transmitted by the mobile phone and stores the basic data in a memory of the eight-electrode body fat scale.

The body fat scale combines measured data such as the measured weight data and the impedance value with the received basic data, and then applies a specific algorithm to accurately calculate a variety of indicator data of the user, for example body fat percentage, water content, muscle mass, and bone mass. The data is of extremely important reference value for people to understand their own health conditions, formulate proper fitness plans, adjust dietary structures, and so on, thereby helping the users conduct health management more scientifically.

It should be noted that the body fat scale in the related art does not have a technical capability of accurately identifying the user and cannot automatically determine an identity of a current user. When the body fat scale is used by multiple users, the users need to manually select basic data of the users on an operation interface of the body fat scale or through a supporting application, to match and associate the basic data of the users with measured data, thereby facilitating subsequent data processing and determination of indicator data.

In actual application scenarios, due to the lack of accurate user identification technology, manual selection operations increase use complexity for the user, reduce use efficiency, and affect user experience. In addition, manual selection operations are likely to cause user errors. Any incorrect selection leads to mismatches between measurement data and the actual user, compromising data accuracy and usability.

In view of this, an embodiment of this application provides a body fat scale and a control method thereof. The body fat scale includes a biometric recognizer, a measurement device, a controller, and a communication module. The biometric recognizer includes a first fingerprint recognizer and a second fingerprint recognizer. The first fingerprint recognizer includes a first fingerprint recognition panel adjustable by an angle; and the second fingerprint recognizer includes a second fingerprint recognition panel that forms the first inclination angle with a vertical plane. The angle of each component conforms to a natural angle at which a finger of the user comes into contact with the body fat scale, which improves the user experience. The controller is configured to: when the number of uses of the body fat scale reaches a preset number and it is detected that a user is in a stable state on the body fat scale, obtain the first initial data of the user; input the first initial data and corresponding historical use data and user preference analysis data into a preset recognition model to determine a target fingerprint recognizer suitable for the user; in response to a fingerprint enrollment operation of the user via the target fingerprint recognizer, obtain the first fingerprint image of the user and determine the first identifier corresponding to the first fingerprint image; and based on the first identifier, establish a matching relationship between the first initial data and basic data corresponding to the first identifier, to learn use habits and the like of the users during use processes of the users to adapt to operational characteristics of different users and quickly and accurately determine identities of the users.

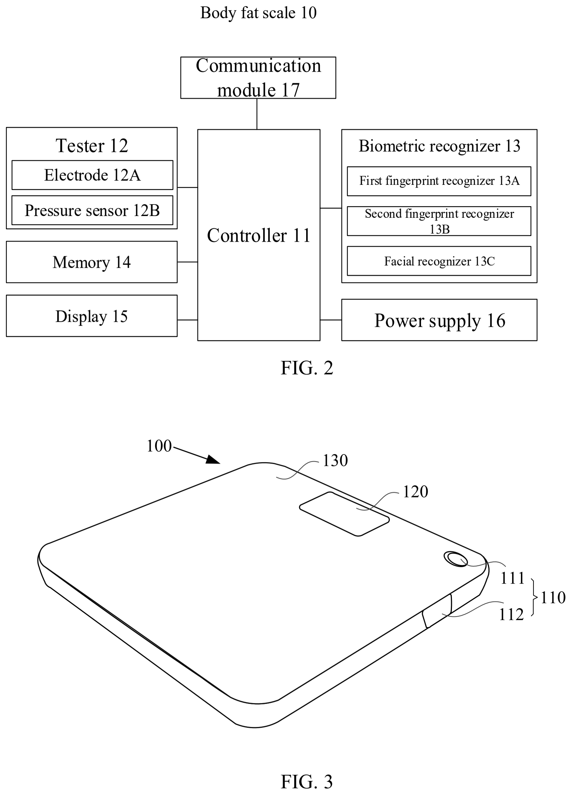

The following describes in detail the body fat scale provided in the embodiments of this application with reference to FIG. 2 to FIG. 9, and illustrates the embodiments of this application through the following multiple exemplary embodiments.

FIG. 2 is a schematic diagram of a system architecture of a body fat scale provided in an embodiment of this application. As shown in FIG. 2, the body fat scale 10 includes a controller 11, a measurement device 12, a biometric recognizer 13, a memory 14, a display 15, a power supply 16, and a communication module 17. The controller 11 is communicatively connected to the measurement device 12, the biometric recognizer 13, the memory 14, the display 15, the power supply 16, and the communication module 17. The measurement device 12 includes an electrode 12A, a pressure sensor 12B, and the like; and the biometric recognizer 13 includes the first fingerprint recognizer 13A, the second fingerprint recognizer 13B, a facial recognizer 13C, and the like.

It should be noted that a connection relationship between the components shown in FIG. 2 are only for illustrative purposes and do not constitute a limitation on the connection relationship between the components in the body fat scale 10. In some other embodiments of this application, the body fat scale 10 may include more or fewer components than those shown in FIG. 2; the body fat scale 10 may include a combination of some components shown in FIG. 2; or the body fat scale 10 may include subcomponents of some components shown in FIG. 2. The components shown in FIG. 2 may be implemented in hardware, software, or a combination of software and hardware.

The controller 11 may include one or more processors. For example, the controller may include a central processing unit (central processing unit, CPU), a graphics processing unit (graphics processing unit, GPU), and the like. Different processors may be independent components, or may be integrated into one processor.

The controller may serve as a nerve center and a command center of the body fat scale 10. The controller can generate an operation control signal based on an instruction operation code and a timing signal, to complete control of instruction fetching and instruction execution.

The measurement device 12 includes an electrode 12A and a pressure sensor 12B. The measurement device 12 is mainly configured to measure the measurement data such as the pressure value and impedance value generated when the user stands on the scale body.

The electrode 12A mainly measures body composition data of the user, for example body fat content through bioelectrical impedance analysis (Bioelectrical Impedance Analysis, BIA) technology. The electrode 12A includes multiple electrode plates, for example four or eight electrode plates.

The electrode plates send a weak and safe electric current to the user. Different tissues and compositions of the body of the user (for example, muscle, fat, and water) have different impedance to the electric current, the adipose tissue has higher electrical resistance, while the muscle tissue, water, and the like have lower electrical resistance. After the electric current passes through the user, the electrode plates then receive a signal fed back, and calculate an impedance value of the user based on transmittance of the electric current in the body of the user. The pressure sensor 12B is configured to measure stable pressure distribution data after the user steps onto the body fat scale, to obtain a corresponding pressure value.

In some embodiments, to improve accuracy of pressure value measurement, pressure sensors 12B include multiple pressure sensors disposed uniformly, and therefore, can not only be configured to accurately obtain pressure distribution data of the user, but also detect pressure distribution when the user performs measurement on the scale body. In this way, a center of gravity of the user can be calculated, which is used to characterize a body balance state and a posture habit of the user, thereby improving measurement accuracy.

The biometric recognizer 13 includes a first fingerprint recognizer 13A and a second fingerprint recognizer 13B, where both the first fingerprint recognizer 13A and the second fingerprint recognizer 13B are configured to collect a fingerprint image of the user, to facilitate determination of the user identity.

It should be understood that in the embodiments of this application, the number of first fingerprint recognizers 13A may be one or more, and the number of second fingerprint recognizers 13B may also be one or more, which are not limited in the embodiments of this application.

The body fat scale provided in the embodiments of this application is equipped with multiple fingerprint recognizers and features a layout designed with full consideration for ergonomics and usage scenarios. Some fingerprint recognizers are disposed on the surface of the body fat scale, while other fingerprint recognizers are hidden on the side surface of the body fat scale, thereby providing more options for users with different use habits. With this design, regardless of the posture of the user when stepping onto the body fat scale, and regardless of a height, a hand shape or a finger characteristic of the user, the user can conveniently and comfortably put the finger on one of the fingerprint recognizers, which greatly improves convenience of obtaining the fingerprint image and optimizes the user experience.

The memory 14 is configured to store basic data, measurement data, a user habit, a preset recognition model, a control algorithm, and the like of the user.

The display 15 is configured to show the basic data, the measurement data, the indicator data, and the like of the user, and can also show a corresponding curve analysis chart and the like of each type of data. The display 15 may be a color display, for example Thin-Film Transistor Liquid Crystal Display, also referred to as a TFT color display.

FIG. 3 is a schematic structural diagram of a body fat scale provided in an embodiment of this application. As shown in FIG. 3, the body fat scale is a four-electrode body fat scale 100. It should be understood that the four-electrode body fat scale 100 includes a controller, a biometric recognizer 110, a measurement device, a display, a memory, a communication module, a power supply, and the like. Ground electrodes in the measurement device include four electrode plates disposed on the first surface 130 of the scale body of the four-electrode body fat scale 100.

The biometric recognizer 110 includes a first fingerprint recognizer 111 and a second fingerprint recognizer 112. The first fingerprint recognizer 111 is disposed on the first surface 130 of the body fat scale, the first fingerprint recognizer 111 includes a first fingerprint recognition panel, and the first fingerprint recognition panel is a panel adjustable by an angle. The second fingerprint recognizer 112 is disposed on a side surface of the body fat scale, the second fingerprint recognizer 112 includes a second fingerprint recognition panel, and there is the first inclination angle between the second fingerprint recognition panel and a vertical plane.

It should be understood that the vertical plane refers to a plane perpendicular to the horizontal plane, that is, a plane in which a plumb line is located. It should be understood that the vertical direction refers to a direction in which any object is perfectly perpendicular to the ground, just like the plumb line. A vertical plane refers to a plane that is orthogonal to the horizontal plane and inclined relative to a front plane and a side plane.

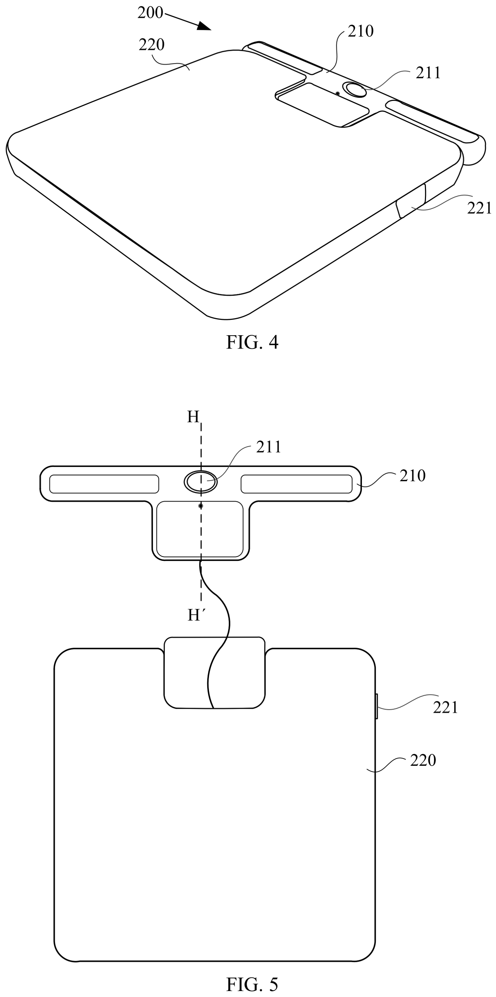

FIG. 4 is a schematic structural diagram of another body fat scale provided in an embodiment of this application. As shown in FIG. 4, the body fat scale is an eight-electrode body fat scale 200. Structurally, the eight-electrode body fat scale 200 includes a handle 210 and a scale body 220.

The eight-electrode body fat scale 200 includes a controller, a biometric recognizer, a measurement device, a display, a memory, a communication module, and a power supply. The electrodes in the measurement device include eight electrode plates, four electrode plates are disposed on the surface of the scale body 220, and the other four electrode plates are disposed on the surface of the handle 210.

The biometric recognizer includes a first fingerprint recognizer 211 and a second fingerprint recognizer 221. The first fingerprint recognizer 211 is disposed on the surface of the handle. It should be understood that when the eight-electrode body fat scale 200 is in a form shown in FIG. 4, the surface of the scale body 220 and the surface of the handle 210 are on the same horizontal plane. In other words, the first fingerprint recognizer 211 is disposed on the first surface of the body fat scale. The first fingerprint recognizer 211 includes a first fingerprint recognition panel, and the first fingerprint recognition panel is a panel adjustable by an angle. The second fingerprint recognizer 221 is disposed on a side surface of the scale body, the second fingerprint recognizer 221 includes a second fingerprint recognition panel, and there is the first inclination angle between the second fingerprint recognition panel and a vertical plane.

For ease of illustration, in all examples in the following embodiments of this application, the body fat scale is the eight-electrode body fat scale. FIG. 5 is a schematic diagram of the body fat scale in FIG. 4 in another form. As shown in FIG. 5, the handle 210 and the scale body 220 of the eight-electrode body fat scale 200 are connected via a retractable cable, and the retractable cable is used for structural connection and communication connection. It should be understood that in the form shown in FIG. 5, the surface of the scale body 220 and the surface of the handle 210 are not on the same horizontal plane, but are respectively located on two intersecting planes. In addition, the user can hold two sides of the handle 210 with both hands and lift the handle up to the front of the face or chest; and at this time, the surface of the handle 210 with the display screen is viewed as the first surface, and the surface of the scale body 220 is viewed as the second surface.

It should be understood that, to configure the first fingerprint recognition panel as the panel adjustable by the angle, the first fingerprint recognizer further includes a universal connection mechanism. The universal connection mechanism is configured to movably connect the first fingerprint recognition panel to the body fat scale, and the first fingerprint recognition panel is provided with a first sensor.

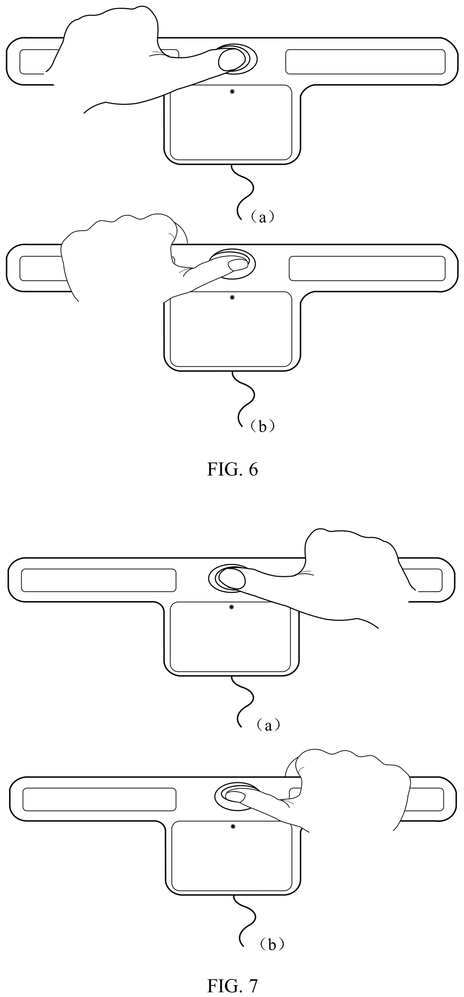

FIG. 6 is a schematic diagram of fingerprint enrollment via the first fingerprint recognizer in a body fat scale provided in an embodiment of this application. Taking fingerprint image enrollment via the left thumb of the user as an example, as shown in figure a in FIG. 6, the left thumb of the user faces toward the first surface of the handle 210; and at this time, the first fingerprint recognition panel is relatively parallel to the first surface. As shown in figure b in FIG. 6, the left thumb of the user faces toward the surface, further away from the display, of the handle 210; and at this time, there is the second inclination angle between the first fingerprint recognition panel and the first surface, which facilitates fingerprint image enrollment of the user. That is, a universal connection mechanism is added to the first fingerprint recognizer to implement angle adjustment of the first fingerprint recognition panel at the vertical angle.

It should be understood that different users have different dominant hands; some users are accustomed to using their left hands, while some other users are accustomed to using their right hands; and therefore, the universal connection mechanism in the first fingerprint recognizer can also adjust the first fingerprint recognition panel to the left or the right in the horizontal direction.

FIG. 7 is a schematic diagram of fingerprint enrollment via the first fingerprint recognizer in a body fat scale provided in an embodiment of this application. Taking fingerprint image enrollment via the right thumb of the user as an example, as shown in figure a in FIG. 7, the right thumb of the user faces toward the first surface of the handle 210; and at this time, the first fingerprint recognition panel is relatively parallel to the first surface. As shown in figure b in FIG. 7, the right thumb of the user faces toward the surface, further away from the display, of the handle 210; and at this time, there is the second inclination angle between the first fingerprint recognition panel and the first surface, which facilitates fingerprint image enrollment of the user. That is, a universal connection mechanism is added to the first fingerprint recognizer to implement angle adjustment of the first fingerprint recognition panel at the vertical angle.

It should be understood that the user can manually rotate the fingerprint recognition panel (for example, the first fingerprint recognition panel or the second fingerprint recognition panel) in each fingerprint recognizer (for example, the first fingerprint recognizer or the second fingerprint recognizer) to an angle suitable for the user.

The universal connection mechanism can be one of structures such as a universal shaft, a spherical joint structure, a cross-shaft universal joint, and a constant velocity joint. With the universal connection structure, the first fingerprint recognition panel can rotate relatively freely within a large angle range, to meet needs of users to enroll fingerprint images through the first fingerprint recognition panel by using different hand gestures, so that different users can enroll fingerprint images through the first fingerprint recognition panel and the corresponding first sensor at angles comfortable for their fingers.

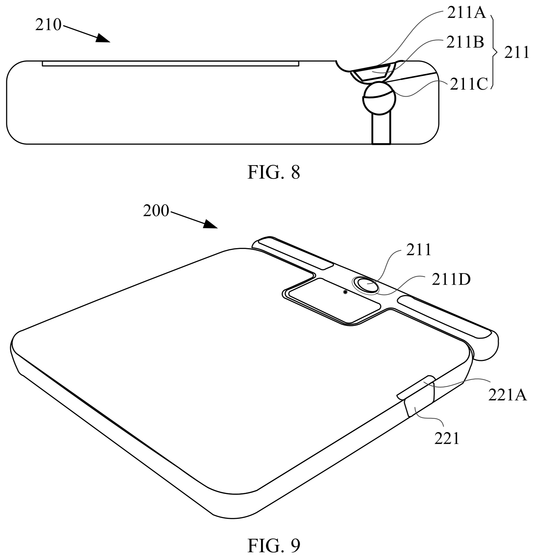

FIG. 8 is a schematic diagram of the first fingerprint recognizer in a body fat scale provided in an embodiment of this application, which is a cross-sectional view at position HH' in FIG. 5. As shown in FIG. 8, the first fingerprint recognizer 211 includes a first fingerprint recognition panel 211A and a spherical joint structure 211C. The spherical joint structure 211C is configured to movably connect the first fingerprint recognition panel 211A to the interior of the body fat scale in an angle adjustable manner. That is, the spherical joint structure 211C is configured to movably connect the first fingerprint recognition panel 211A to the interior of the handle in an angle adjustable manner.

It should be understood that the first fingerprint recognizer may further include a fixing base, a rotating shaft, and a first fingerprint recognition panel. The fixing base is configured to fix the entire apparatus onto the body fat scale, and the rotating shaft connects the fixing base to the main body of the fingerprint recognition panel, so that the panel can rotate around the shaft.

In some embodiments, the universal connection mechanism further includes an angle sensor, and the angle sensor is configured to accurately measure and feed back a rotation angle of the first fingerprint recognition panel.

In some embodiments, the universal connection mechanism further includes an angle sensor and a motor. The angle sensor is configured to accurately measure and feed back the rotation angle of the first fingerprint recognition panel, and the motor is configured to drive the universal connection mechanism to rotate based on an angle instruction sent by the controller.

It should be understood that the universal connection mechanism is driven via a motor to drive the fingerprint recognition panel to rotate, thereby implementing automatic angle adjustment. With this structure, multi-angle adjustment within a relatively wide range can be implemented, thereby meeting needs of different users (with, for example, different heights and use habits) to enroll fingerprints in comfortable postures.

In the embodiments of this application, the first fingerprint recognition panel is designed to be adjustable by an angle. In this way, the users can easily put their fingers on the fingerprint recognizer, regardless of whether the first fingerprint recognizer is disposed on the surface of the body fat scale in FIG. 3 or on the surface of the handle of the body fat scale in FIG. 4, which reduces difficulty and time of the operation and improves efficiency of obtaining the fingerprint image.

As shown in FIG. 3 and FIG. 4, the second fingerprint recognizer is disposed on the side surface of the body fat scale or the side surface of the scale body of the body fat scale. For convenience of the user, there is the first inclination angle between the second fingerprint recognition panel in the second fingerprint recognizer and the vertical plane. It should be understood that the first inclination angle can be set via inclination toward the body fat scale or a center of the scale body, to match an inward-tilting angle of the finger when the finger of the user hangs down naturally, so that the user can easily put the finger on the fingerprint recognizer, thereby reducing the difficulty and time of the operation and improving the efficiency of obtaining the fingerprint image.

In some embodiments, the first inclination angle can also be set via inclination away from the body fat scale or the scale body, so that young and/or short users can also easily put their fingers on the fingerprint recognizer, thereby reducing the difficulty and time of the operation and improving the efficiency of obtaining the fingerprint image.

The first sensor 211B is disposed in the first fingerprint recognition panel 211A. It should be understood that the first sensor 211B may be an optical fingerprint sensor or a capacitive fingerprint sensor.

In some embodiments, to improve the recognition speed and accuracy of the fingerprint image, the first sensor is an integrated sensor, including the optical fingerprint sensor and the capacitive fingerprint sensor, namely, a combination of the optical fingerprint sensor and the capacitive fingerprint sensor. The optical fingerprint sensor can quickly obtain an overall image of a fingerprint, while the capacitive fingerprint sensor can capture detailed features of the fingerprint more accurately, for example ridges and valleys of the fingerprint. The integration of multiple types of sensors can improve both the recognition speed and accuracy of the fingerprint. Even when the finger has slight stains or is damp, the fingerprint image can still be quickly and accurately obtained.

In some embodiments, the body fat scale can also obtain environmental information (for example, temperature, humidity, and light intensity) through an application on a terminal device. Therefore, an adaptive fusion algorithm can be used to dynamically adjust weights of data of the two types of sensors based on different environmental conditions (for example, the humidity and the light intensity) and fingerprint features (for example, a dryness level and clarity of fingerprint patterns) of the user. For example, in an environment with high humidity, the weight of the data of the capacitive fingerprint sensor is increased, because image quality of the optical fingerprint sensor may decrease in a humid environment. The weights are continuously adjusted, so that fused fingerprint features are more accurate, thereby improving the recognition accuracy.

The first fingerprint recognizer further includes a first indicator light, and the first indicator light is disposed on the first surface of the body fat scale. When emitting light in a preset mode, the first indicator light is configured to inform the user that the corresponding first fingerprint recognizer is the fingerprint recognizer that is most suitable for use. The first indicator light constantly emits light or flashes to instruct the user to use the first fingerprint recognizer to enroll the fingerprint.

For example, the first indicator light can be disposed on the periphery of the first fingerprint recognition panel, or on the periphery of the first fingerprint recognizer on the first surface. The first indicator light can be annular, dot-shaped, or strip-shaped. A position and a shape of the first indicator light is not limited in the embodiments of this application.

FIG. 9 is a schematic structural diagram of still another body fat scale provided in an embodiment of this application. As shown in FIG. 9, the first indicator light 211D of the first fingerprint recognizer 211 is disposed on the periphery of the first fingerprint recognizer 211 and configured to be annular.

In some embodiments, the first fingerprint recognizer further includes an LED light source. The LED light source emits red light and infrared light, which penetrate the skin and blood vessels. When blood flows, oxyhemoglobin and deoxyhemoglobin absorb the two types of light in different proportions. After the light is partially absorbed by the blood vessels, remaining light is received by the first sensor in the first fingerprint recognizer. In addition, the signal collection and processing circuit of the first sensor is optimized to measure a change in a pulse wave and an absorption degree of light.

In the first fingerprint recognition panel of the first fingerprint recognizer, LEDs that emit the red light and infrared light are integrated to serve as a light source for heart rate measurement.

It should be understood that the first sensor includes the optical fingerprint sensor and the capacitive fingerprint sensor. To be specific, the first fingerprint recognizer has a photoelectric detection function for capturing the fingerprint image. After an LED light source is added, the existing photoelectric sensor can be used to detect changes in absorption and reflection of the red light and infrared light as the red light and infrared light pass through the blood vessels in the finger, thereby obtaining a photoplethysmography signal related to the heart rate.

It should be understood that the second fingerprint recognition panel is provided with a second sensor. The second sensor may be the optical fingerprint sensor or the capacitive fingerprint sensor; or the second sensor may include both the optical fingerprint sensor and the capacitive fingerprint sensor.

The second fingerprint recognizer further includes a second indicator light. When emitting light in a preset mode, the second indicator light is configured to inform the user that the corresponding second fingerprint recognizer is the fingerprint recognizer that is most suitable for use. The second indicator light constantly emits light or flashes to instruct the user to use the second fingerprint recognizer to enroll the fingerprint. The position and form of the second indicator light are not limited in the embodiments of this application.

It should be understood that the second fingerprint recognizer is disposed on the side surface of the body fat scale and therefore, is not easy to find from an angle of view of the user (for example, from the angle of view of the user when looking down to check). Therefore, the second indicator light is disposed on the surface of the body fat scale and corresponds to the second fingerprint recognizer on the side surface. As shown in FIG. 9, the second indicator light 221A in the second fingerprint recognizer 221 is disposed on the surface of the scale body, to facilitate check of the user. In addition, the second indicator light 221A is connected to the second fingerprint recognizer and is strip-shaped.

In some embodiments, the second fingerprint recognizer further includes an LED light source. For details, refer to the above description of the LED light source in the first fingerprint recognizer. Details are not described herein again.

In some embodiments, second fingerprint recognizers may be disposed in a circle around the body fat scale, and each second fingerprint recognizer is correspondingly equipped with the second indicator light. When it is determined that the target fingerprint recognizer is one of the second fingerprint recognizers, the corresponding second indicator light automatically emits light, to instruct the user to quickly put a finger at a correct position, thereby obtaining a fingerprint image more rapidly. In addition, different colors of lights can also be set for different users to improve customized experience.

It should be understood that an LED light source is added to each fingerprint recognizer to monitor the heart rate of the user. There are differences between heart rates of different users, and therefore, on the premise that the fingerprint recognition function is not affected, the monitoring of the heart rate data of the user is added to obtain historical use data of the user and optimize the preset recognition model and the like.

In other embodiments, the biometric recognizer further includes a facial recognizer, and the facial recognizer is disposed on the surface of the handle. A camera disposed at the top end of the display in the handle can accurately capture features of the face of the user. For example, with infrared fill light technology, the camera can clearly and accurately capture the features of the face of the user even in dimly lit environments.

When the user stands on the body fat scale, the camera is activated to capture the facial image of the user. With a deeply optimized face recognition algorithm embedded into the controller, the identity of the user can be quickly determined, which forms a dual security mechanism together with the fingerprint recognizer, thereby greatly improving recognition accuracy and convenience of the identity. Regardless of whether the user still has a drowsy look in the morning or sweats profusely after exercise, the facial recognizer can work stably, to provide the user with smooth use experience and easily achieve accurate data matching when multiple users share the body fat scale.

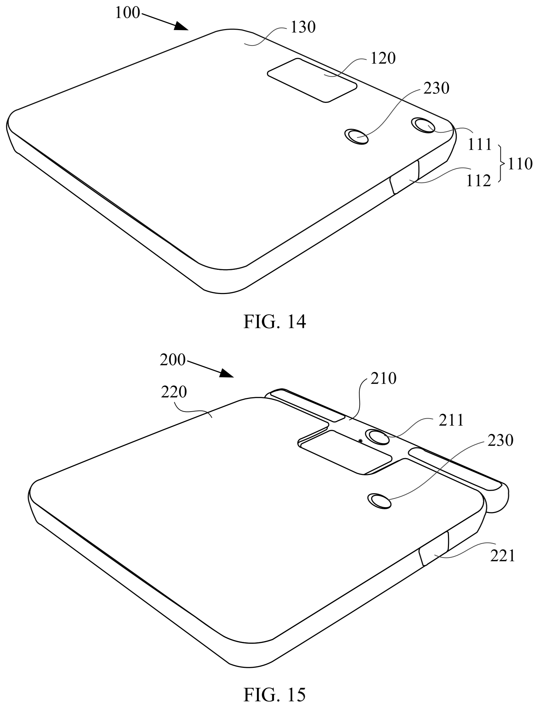

As shown in FIG. 14 and FIG. 15, in some embodiments, the biometric recognizer further includes a toe print recognizer 230. The toe print recognizer 230 is disposed on the first surface of the body fat scale, specifically on the upper surface of the scale body 220, so that when the user normally uses the body fat scale for weight measurement, a toe can naturally touch and press against a sensing area of the toe print recognizer. An installation method of integrating the toe print recognizer 230 with the scale body 220 is used, and data interaction is implemented for circuit connection of the toe print recognizer 230 through a main control board inside the body fat scale. This toe print recognizer 230 is compatible with two types of body fat scale products: the four-electrode body fat scale and the eight-electrode body fat scale, without a need to readjust a layout of the main electrodes of the body fat scale.

When the user stands barefoot on the upper surface of the scale body 220 and maintains a stable posture, a toe part of the user comes into effective contact with the sensing area of the toe print recognizer 230. Under the pressure caused due to the body weight of the user, the toe print recognizer 230 starts a working state, collects texture features of the surface of the toe of the user through a built-in sensor, and further generates corresponding toe print image data. Then the toe print recognizer transmits the collected toe print image data to the controller of the body fat scale. The controller performs preprocessing and feature extraction on the image data, generates toe print feature information that can uniquely identify the user, and allocates the corresponding first identifier to the feature information. The first identifier is used for matching and comparison with a stored user profile in a subsequent identity determination process, thereby realizing a user identity determination function based on the toe print.

In some embodiments, the toe print recognizer 230 includes the third fingerprint recognition panel adjustable by an angle and the third sensor. The third fingerprint recognition panel is movably connected to the body fat scale via a universal connection mechanism, so that the third fingerprint recognition panel can be tilted, deflected, or leaned by an angle within a certain range, to adapt to changes in toe pressing angles caused due to a difference in foot postures of different users during the use of the body fat scale, thereby ensuring good contact between the toes and the third fingerprint recognition panel and improving quality of the collected toe print image. The third sensor is integrated onto the third fingerprint recognition panel. The third sensor is configured to detect the texture features of the surface of the toe. When the toe of the user presses against the third fingerprint recognition panel, the third sensor collects the corresponding toe print image data and transmits the data to the controller of the body fat scale for subsequent feature extraction and identity determination processing.

In some embodiments, the third sensor includes an optical fingerprint sensor and a capacitive fingerprint sensor. The optical fingerprint sensor and the capacitive fingerprint sensor are combined. The optical fingerprint sensor can quickly obtain an overall image of a fingerprint, while the capacitive fingerprint sensor can capture detailed features of the fingerprint more accurately, for example ridges and valleys of the fingerprint. The integration of multiple types of sensors can improve both the recognition speed and accuracy of the fingerprint. Even when the finger has slight stains or is damp, the fingerprint image can still be quickly and accurately obtained.

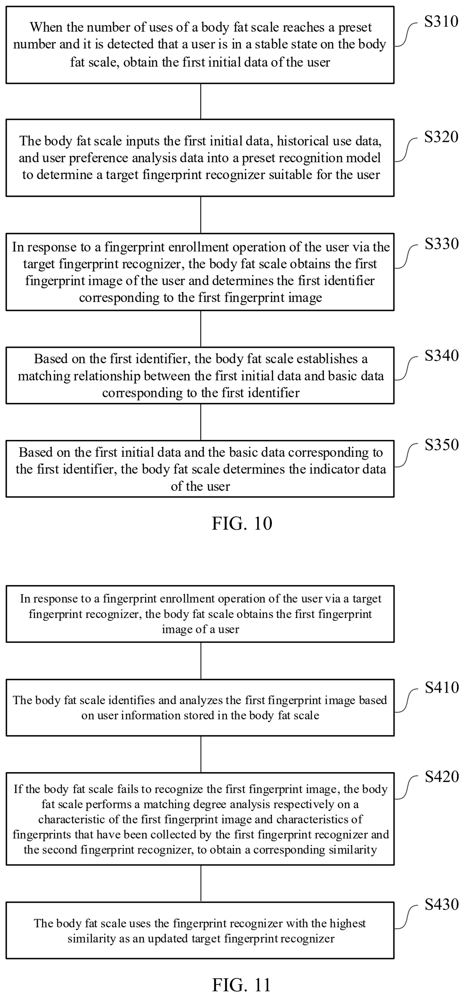

The following describes in detail the body fat scale controlling method provided in this embodiment of this application with reference to FIG. 10 and FIG. 11. It should be noted that if substantially identical results are achieved, the method in application is not limited to the order of the procedure shown in FIG. 10 and FIG. 11.

The embodiment of this application further provides a body fat scale controlling method, applied to the foregoing body fat scale. FIG. 10 is a schematic flowchart of a body fat scale controlling method provided in an embodiment of this application. As shown in FIG. 10, the body fat scale controlling method includes the following steps:
S310. When the number of uses of the body fat scale reaches a preset number and it is detected that a user is in a stable state on the body fat scale, obtain the first initial data of the user.

The first initial data includes bioimpedance data and pressure distribution data. It should be understood that the bioimpedance data is obtained via an electrode, and the pressure distribution data is obtained via the pressure sensor.

The preset number is determined by developers of the body fat scale during research and development. For example, the preset number can be 10 or 20. It should be understood that the preset number is controlled to be a minimum value that meets a requirement for developing the learning capability, to improve the user experience.

It should be understood that since the first use, the body fat scale remains in a learning mode until the number of uses reaches the preset number, and obtains data such as the historical use data, the user preference analysis data, and the like of the users by learning about different users of the body fat scale.

A first-use guidance process is set up in the body fat scale. During the first use of the body fat scale, a communication connection between the mobile phone and the eight-electrode body fat scale is established via an application on the mobile phone, and the user is required to input basic data such as gender, age, and height into the application to facilitate subsequent better data matching and analysis. In addition, the body fat scale instructs the user to use each fingerprint recognizer in sequence to enroll fingerprint images, and the body fat scale records the historical use data of the user such as fingerprint images and a corresponding use status of the fingerprint recognizer, and analyzes features in the fingerprint images collected by different fingerprint recognizers, including information such as clarity of the fingerprint image, fingerprint patterns, and the number of feature points. For example, on a body fat scale equipped with three fingerprint recognizers (for example, one first fingerprint recognizer and two second fingerprint recognizers), the user enrolls the fingerprint image into each fingerprint recognizer as prompted, with a total of 3 enrollments of fingerprint images. Features in the fingerprint image collected each time are analyzed.

During the period from the second use to the N^{th} use (the preset number of uses is N+1), the body fat scale records the use frequency of the fingerprint recognizer and the like via the historical use data, which facilitates learning of a user habit of using a specific fingerprint recognizer to enroll the fingerprint, and pressure distribution data and heart rate data of the user during the subsequent process of optimizing the preset recognition model.

It should be understood that the body fat scale initializes each sensor in each fingerprint recognizer before each use. For example, the body fat scale sets parameters such as exposure time and image resolution for the optical fingerprint sensor; and the body fat scale calibrates a sensing capacitance range and the like for the capacitive fingerprint sensor.

It should be noted that, by using multiple pressure sensors evenly distributed inside the body fat scale, it is determined that the user is in the stable state on the body fat scale. When the user steps onto the body fat scale, the pressure sensors start working. If changes in pressure values detected by each pressure sensor fall within an extremely small range (for example, fluctuate within ±5%), and the overall pressure distribution is relatively uniform and stable within a certain period of time (for example, 1-3 seconds), it can be determined that the posture of the user is stable. For example, when the user stands stably on both feet, pressure distribution data measured by the pressure sensors on the left and right sides as well as the front and rear positions remains stable, which indicates that the posture of the user is steady.

In some embodiments, an acceleration sensor can also be built in the body fat scale, which can detect a change in acceleration of the body fat scale in each direction. When the user stands on the scale, the acceleration sensor collects data in real time. When acceleration changes in the three axes (X, Y, and Z) all approach zero and are maintained for a certain period of time (for example, 1-2 seconds), this means that the body fat scale is in a stationary state, and it can be determined that the user is in the stable state. For example, the body of the user stops swaying, the body fat scale shows no displacement or tilt, and the data from the acceleration sensor becomes stable.

In other embodiments, the data from the pressure sensor and the acceleration sensor can be integrated. Weighted calculation and logical judgment is performed by analyzing the stability of the pressure distribution data as well as the acceleration changes. In-depth analysis of multi-source data is performed through a preset algorithm, and therefore, it can be more accurately determined whether the user is in the stable state, thereby effectively avoiding potential misjudgment caused by a single sensor and ensuring accuracy of the measurement result.

S320. The body fat scale inputs the first initial data, historical use data, and user preference analysis data into a preset recognition model to determine a target fingerprint recognizer suitable for the user.

It should be understood that the historical use data and user preference analysis data are obtained, analyzed, and then stored when the number of uses of the body fat scale has not yet reached the preset number.

Therefore, after the first initial data is obtained, the first initial data, the historical use data, and the user preference analysis data are collectively input into the preset recognition model as input data, and in this way, the target fingerprint recognizer suitable for the user can be obtained. The target fingerprint recognizer is the first fingerprint recognizer or the second fingerprint recognizer.

It should be understood that the preset recognition model in step 320 is a pre-trained model, and is optimized by using the use data corresponding to a case that the number of uses has not reached the preset number.

The preset recognition model is a machine learning model and can be constructed by using algorithms such as a decision tree and a neural network. Taking the neural network model as an example, the neural network model includes an input layer, a hidden layer, and an output layer. The input layer is responsible for receiving the first initial data, the historical use data, and the user preference analysis data. After the data is subjected to complex calculations and feature extraction by numerous neurons in the hidden layer, the result of the target fingerprint recognizer suitable for the user is finally output by the output layer.

The body fat scale learns the use habit, a fingerprint placement method, fingerprint enrollment preference, and the like of the user via the preset recognition model, and automatically adjusts a parameter and a policy of the preset recognition model to adapt to operating characteristics of different users, thereby obtaining the fingerprint image of the user faster and more accurately in subsequent use.

The preset recognition model deployed on the body fat scale is a pre-trained model. Input data of the preset recognition model includes at least the first initial data, the historical use data, and the user preference analysis data, and may also include the heart rate data and the like.

During the research and development process of the body fat scale, numerous samples are collected, which include different types of data and corresponding annotations of target fingerprint recognizers. The sample data is input into the preset recognition model. The preset recognition model processes the input data based on a rule of the selected algorithm and outputs a prediction result. Then the prediction result is compared with the actual annotation, and a loss function is calculated to measure a difference between the two parameters. Based on a value of the loss function, a backpropagation algorithm is used to adjust connection weights of neurons in each layer of the preset recognition model, to reduce the loss value. After multiple rounds of iterative training, the preset recognition model is continuously optimized until indicators such as satisfactory accuracy and recall rates are achieved on a validation set, so that the preset recognition model can be used to determine the target fingerprint recognizer suitable for the user.

It should be understood that optimization is performed by using the use data corresponding to a case that the number of uses has not reached the preset number. That is, the model is optimized when the number of uses of the body fat scale has not reached the preset number and it is detected that the user is in the stable state on the body fat scale. Before step 310, the body fat scale controlling method also include the following operations 1 to 3:

Operation 1: The body fat scale obtains the second fingerprint image and the second initial data of the user, where the second fingerprint image is an image collected via the first fingerprint recognizer and/or the second fingerprint recognizer.

It should be understood that if the body fat scale is used for the first time, the second fingerprint image includes an image collected by the first fingerprint recognizer and an image collected by the second fingerprint recognizer.

If the body fat scale is used for the second time to the N^{th} time (the preset number of uses is N+1), the second fingerprint image includes the image collected by the first fingerprint recognizer and/or the image collected by the second fingerprint recognizer.

For a purpose of differentiation, when the number of uses of the body fat scale has not reached the preset number, the measurement data obtained through the measurement device is referred to as the second initial data; and when the number of uses of the body fat scale has reached the preset number, the measurement data obtained through the measurement device is referred to as the first initial data.

Therefore, the second initial data includes bioimpedance data and pressure distribution data. It should be understood that the bioimpedance data is obtained via an electrode, and the pressure distribution data is obtained via the pressure sensor.

Operation 2: The body fat scale analyzes the second fingerprint image and the second initial data to obtain the historical use data and user preference analysis data of the user.

When the number of uses of the body fat scale has not reached the preset number, the preset recognition model is optimized. Therefore, in this process, the second fingerprint image and the second initial data need to be analyzed. Firstly, during each use, the corresponding fingerprint recognizer, use time, and the like are recorded, and a feature of the second fingerprint image is extracted to identify unique information of the user, for example clarity of a fingerprint image, a fingerprint pattern, and the number of feature points, thereby facilitating optimization of the preset recognition model to be more suitable for the user based on the second fingerprint image. Further, the historical use data and the user preference analysis data of the user are obtained.

The current second fingerprint image is associated with information such as the second initial data and the corresponding fingerprint recognizer, and is compared, integrated, and statistically analyzed with data accumulated based on previous identifications of the same fingerprint, and in this way, the historical use data of the user can be obtained, which includes information such as use frequency during different time periods.

During multiple use processes of the user, various preference settings of the user are recorded, for example a frequently used fingerprint recognizer and adaptivity of the user to automatic angle adjustment. Data obtained each time is deeply integrated with the stored historical use data. Data in different periods is compared and correlatively analyzed, so that the body fat scale can further learn a behavior pattern and preference of the user, analyzes tendency of the user in choosing a body fat scale function and a fingerprint recognizer, and improves the user preference analysis data, thereby obtaining comprehensive and detailed user preference analysis data.

Operation 3: The body fat scale optimizes the preset recognition model based on the second fingerprint image, the second initial data, the historical use data, and the user preference analysis data, to obtain an optimized preset recognition model.

In daily use scenarios of the body fat scale, intelligence of the body fat scale is gradually improved as the number of times of using the body fat scale by the user increases. Each time the user completes a measurement, the body fat scale collects the second fingerprint image to accurately determine the identity of the user.

The body fat scale uses the second fingerprint image, the second initial data, historical use data, and user preference analysis data together as input data to perform a comprehensive optimization of the preset recognition model. During continuous iterative operations, the preset recognition model gradually adjusts its internal parameters to improve recognition accuracy of an individual characteristic and a behavioral habit of the user. In this way, the optimized preset recognition model is obtained, to further provide a more accurate target fingerprint recognition model for the user in the subsequent use process and deliver a customized service to the user.

In some embodiments, after determining the target fingerprint recognizer suitable for the user, the controller generates prompt information based on the target fingerprint recognizer, and the prompt information is used to instruct the user to enroll a fingerprint via the target fingerprint recognizer. It should be understood that the prompt information can be presented in different forms to achieve a purpose of prompting the user.

In some embodiments, when the prompt information indicates that the first fingerprint recognizer is the target fingerprint recognizer, the first indicator light is driven to emit light based on a preset mode; or when the prompt information indicates that the second fingerprint recognizer is the target fingerprint recognizer, the second indicator light is driven to emit light based on the preset mode, where the preset mode includes a constant-on mode or a flashing mode.

In some embodiments, the body fat scale further includes an audio transceiver with a speaker. The prompt information can also be in a voice form to prompt the user to put a finger on the specified target fingerprint recognizer, and instruct the user to put the finger correctly to obtain the optimal recognition effect. For a user with poor eyesight or a user unfamiliar with the operation, a voice prompt can greatly facilitate obtaining of the fingerprint image. In other embodiments, the audio transceiver further includes a microphone. A corresponding voice recognition model is added to the controller, to implement voice interaction with the user.

In some embodiments, the prompt "The XX fingerprint recognition area is the optimal use area" is displayed on the display of the body fat scale or the corresponding application. It should be understood that the "XX fingerprint recognition area" here corresponds to the target fingerprint recognizer on the body fat scale, and the text for prompting the user is easy to understand.

It should be understood that during use, the preset recognition model continuously learns and upgrades, constantly learns changes in the fingerprint feature, the use habit, the preference, and the like of the user, dynamically optimizes the preset recognition model, and updates a recommendation result of the optimal fingerprint recognizer.

S330. In response to a fingerprint enrollment operation of the user via the target fingerprint recognizer, the body fat scale obtains the first fingerprint image of the user and determines the first identifier corresponding to the first fingerprint image.

The target fingerprint recognizer is the first fingerprint recognizer or the second fingerprint recognizer, and the user is instructed to enroll the fingerprint image via the target fingerprint recognizer.

It should be noted that the first fingerprint image is identified and analyzed based on user information stored in the body fat scale. If the first fingerprint image is recognized, the first identifier corresponding to the first fingerprint image is determined.

It should be understood that the terminal device sends the basic data of the user to the body fat scale, matches the basic data with the second fingerprint image obtained when the number of uses has not reached the preset number, and stores the basic data in the body fat scale. To facilitate differentiation between different users, an identifier can be set for each user. When it is determined that the first fingerprint image belongs to a user whose data has been stored, the identifier of the user is used as the first identifier corresponding to the first fingerprint image.

In some embodiments, after the first fingerprint image is enrolled, during identification of the first fingerprint image, the user can be informed of an identification status in different forms. For example, a progress bar is displayed on the display of the body fat scale, or a voice prompt "The fingerprint is being identified, please wait" is pushed through the application. If the first fingerprint image is successfully recognized, the message "Recognition successful, measuring indicator data now" can be displayed; and if the identification fails, a specific reason prompt can also be provided, for example "The fingerprint is blurred, please put the figure again" or "This fingerprint is unregistered, please register first".

S340. Based on the first identifier, the body fat scale establishes a matching relationship between the first initial data and basic data corresponding to the first identifier.

It should be understood that the first identifier is used to identify the user. Further, based on the first identifier, a matching relationship can be established between the first initial data and the basic data corresponding to the first identifier, which facilitates subsequent data analysis and determination of the indicator data.

S350. Based on the first initial data and the basic data corresponding to the first identifier, the body fat scale determines the indicator data of the user.

The body fat scale controlling method provided in this embodiment of this application includes: when the number of uses of the body fat scale reaches a preset number and it is detected that a user is in a stable state on the body fat scale, obtaining the first initial data of the user; inputting the first initial data and corresponding historical use data and user preference analysis data into a preset recognition model to determine a target fingerprint recognizer suitable for the user; in response to a fingerprint enrollment operation of the user via the target fingerprint recognizer, obtaining the first fingerprint image of the user and determining the first identifier corresponding to the first fingerprint image; and based on the first identifier, establishing a matching relationship between the first initial data and basic data corresponding to the first identifier, to learn use habits and the like of the users during use processes of the users to adapt to operational characteristics of different users and quickly and accurately determine identities of the users.

In some embodiments, after the first fingerprint image is obtained, it is possible that the first fingerprint image is unrecognized during the process of identifying and analyzing the first fingerprint image. A fault tolerance rate of the solution, user experience, and the like need to be improved. FIG. 11 is a schematic flowchart of analysis in a body fat scale controlling method provided in an embodiment of this application. As shown in FIG. 11, after the body fat scale obtains the first fingerprint image of the user in response to the fingerprint enrollment operation of the user via the target fingerprint recognizer in step 330, the method further includes the following steps.

S410. The body fat scale identifies and analyzes the first fingerprint image based on user information stored in the body fat scale.

It should be understood that the terminal device sends the basic data of the user to the body fat scale, matches the basic data with the second fingerprint image obtained when the number of uses has not reached the preset number, and stores the basic data in the body fat scale. To facilitate differentiation between different users, an identifier can be set for each user. When it is determined that the first fingerprint image belongs to a user whose data has been stored, the identifier of the user is used as the first identifier corresponding to the first fingerprint image; and when it is determined that the first fingerprint image does not belong to the user whose data has been stored, this indicates that the first fingerprint image is unrecognized.

S420. If the body fat scale fails to recognize the first fingerprint image, the body fat scale performs a matching degree analysis respectively on a characteristic of the first fingerprint image and characteristics of fingerprints that have been collected by the first fingerprint recognizer and the second fingerprint recognizer, to obtain a corresponding similarity.

It should be understood that when the fingerprint image currently enrolled by the user is unrecognized, a process of switching the fingerprint recognizer is started. The database that contains the fingerprint features of the user and has been recorded in the previously optimized preset recognition model is quickly retrieved from the memory. The database stores in detail a variety of feature information of the user when each fingerprint recognition area collects the fingerprint, including the clarity of fingerprint pattern, the number of feature points, and the like.

A similarity matching algorithm is used to compare a feature of the current unrecognized fingerprint image (namely, the first fingerprint image) with features of the fingerprints collected by fingerprint recognizers one by one, and calculate a similarity between the fingerprint collected by each fingerprint recognizer and the first fingerprint image. For example, the Euclidean distance algorithm or cosine similarity algorithm is used to quantitatively evaluate a similarity between the features of the fingerprints.

After the similarities are calculated, the fingerprint recognizers are sorted in a descending order of similarity scores to obtain similarity ranking.

S430. The body fat scale uses the fingerprint recognizer with the highest similarity as an updated target fingerprint recognizer.

It should be understood that the fingerprint recognizer with the highest similarity score in the similarity ranking is the fingerprint recognizer that best matches the fingerprint feature of the current user, and in this way, the target fingerprint recognizer is updated.

In addition, corresponding prompt information is generated to inform the user by sending, for example, a clear prompt to the user via the display of the body fat scale or the application connected thereto. The prompt content may include information about the fingerprint recognizer recommended for switching, and can also briefly explain a reason for the recommendation, for example, "The feature of your fingerprint pattern has the highest matching degree with the collected data of the XX fingerprint recognizer. It is recommended to switch to the XX fingerprint recognizer for identification".

It should be understood that after the target fingerprint recognizer is updated, the foregoing step 330 is executed again.

To ensure that the user can conveniently switch between the fingerprint recognizers, corresponding interactive guidance can also be provided on a prompt interface. For example, an image is generated on the application, and the image is used to prompt the user of a location corresponding to the target fingerprint recognizer. On the display of the body fat scale, the user is instructed to put the finger on the recommended fingerprint recognizer through a lighting effect of the indicator light. In addition, the operation data of the user during each switch of the fingerprint recognizer can also be recorded, which facilitates further optimization of the preset recognition model and continuous improvement of recognition efficiency and accuracy.

It should be understood that in some embodiments, the universal connection mechanism in the first fingerprint recognizer further includes an angle sensor and a motor. In this case, after the target fingerprint recognizer suitable for the user is determined in step 320, the method further includes the following operations 1 to 3:
Operation 1: If the target fingerprint recognizer is the first fingerprint recognizer in the body fat scale, analyze historical use data to determine a target angle of the first fingerprint recognition panel.

The historical use data includes use frequency of the first fingerprint recognizer.

For details, refer to the above description of obtaining and analysis of the historical use data. Details are not described herein again.

Operation 2: When the user enrolls the fingerprint, the body fat scale obtains an initial angle corresponding to the first fingerprint recognition panel via the angle sensor. The angle sensor is configured to accurately measure and feed back a rotation angle of the first fingerprint recognition panel.

Operation 3: The body fat scale controls the motor to adjust the angle of the first fingerprint recognition panel to a target angle.

For the first fingerprint recognition panel adjustable by an angle, based on a position and an angle at which the user puts the finger, the panel angle can be adjusted via a motor drive algorithm embedded in the controller. That is, the first fingerprint recognition panel corresponding to the first sensor is adjusted to the corresponding angle to which the user is accustomed, so that the first sensor better fits the finger of the user.

In some embodiments, a difference between the angle at which the user puts the finger and the current angle of the first fingerprint recognition panel can also be measured, and the first fingerprint recognition panel is adjusted based on this difference. For example, when it is detected that the difference between the angle at which the user puts the finger and the current angle of the panel exceeds 15 degrees, an angle value by which the current angle needs to be adjusted is calculated, and the motor is controlled to adjust the panel to an appropriate angle, to improve quality of the collected fingerprint and a success rate of recognition.

When the motor is controlled to adjust the angle of the first fingerprint recognition panel, the user can be informed that the angle adjustment is in progress through the flashing of the first indicator light or a voice prompt. For example, a voice prompt "Adjusting the angle of the fingerprint recognition panel, please keep your finger steady" is issued. In addition, the first indicator light around the fingerprint recognition area flashes rapidly, to inform the user of the current operation.

In the body fat scale controlling method provided in this embodiment of this application, the universal connection mechanism can be further driven via a motor to drive the fingerprint recognition panel to rotate, thereby implementing automatic angle adjustment. With this structure, multi-angle adjustment within a relatively wide range can be implemented, thereby meeting needs of different users (with, for example, different heights and use habits) to enroll fingerprints in comfortable postures.

In some embodiments, the body fat scale can also obtain heart rate data through the LED light source and the first sensor. That is, the first initial data also includes heart rate data, and the second initial data also includes heart rate data. On the premise that the fingerprint recognition function is not affected, a dedicated heart rate signal processing algorithm can be added to the controller, to subject a collected photoplethysmography signal to processing such as filtering, amplification, and feature extraction, thereby calculating the heart rate data of the user.

It should be understood that the heart rate data can be used to obtain the historical use data of the user.

It should be understood that for the body fat scale equipped with the LED light source, the heart rate data is combined with the corresponding preset recognition model during a training process. Because more input data can cover more features and changes, the preset recognition model can learn more complex and subtle patterns and rules, which reduces a risk of overfitting and enhances a generalization capability for unknown data, thereby improving accuracy of the preset recognition model. In addition, during the optimization and use of the preset recognition model, the heart rate data is used as the input data to optimize the preset recognition model, so that the optimized preset recognition model more accurately outputs a result of the target fingerprint recognizer suitable for the user.

Therefore, in step 320, the heart rate data is also used as the input data to input into the preset recognition model to determine the target fingerprint recognizer suitable for the user.

In some embodiments, the body fat scale controlling method further includes: when the finger of the user is close to the fingerprint recognizer but has not yet come into full contact with the fingerprint recognizer, performing preliminary analysis on a weak signal captured by a sensor corresponding to the fingerprint recognizer (the first sensor or the second sensor), to determine whether the fingerprint is valid, and starting relevant recognition preparation work in advance, so that when the finger comes into full contact with the fingerprint recognizer, the obtaining and recognition of the fingerprint image can be completed more quickly.

It should be understood that the foregoing descriptions are examples of corresponding application scenarios and do not impose any limitation on application scenarios of this application.

It should be understood that the foregoing examples are provided to assist persons skilled in the art in understanding the embodiments of this application, and are not intended to limit the embodiments of this application to the illustrated specific values or specific scenarios. Persons skilled in the art apparently may make various equivalent modifications or changes based on the examples provided above, and such modifications or changes also fall within the scope of the embodiments of this application.

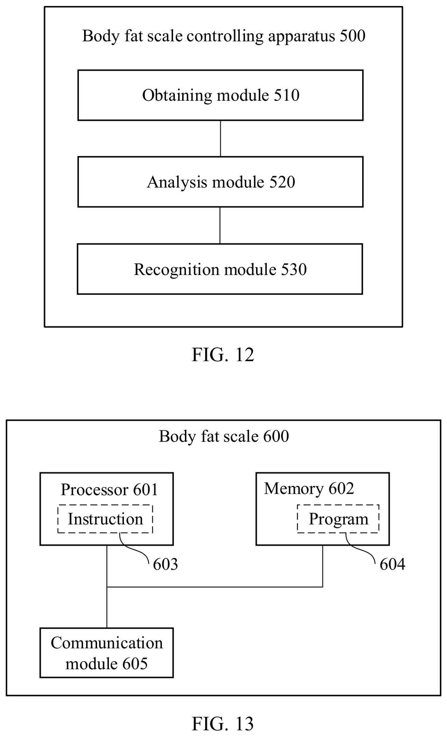

The body fat scale and the body fat scale controlling method in the embodiments of this application are described in detail above with reference to FIG. 2 to FIG. 11. The following describes in detail an apparatus embodiment of this application with reference to FIG. 12. It should be understood that the control apparatus in this embodiment of this application can perform various body fat scale controlling methods in the foregoing embodiments of this application. That is, for the specific working processes of the various products below, refer to the corresponding processes in the foregoing method embodiments.

FIG. 12 is a schematic diagram of a body fat scale controlling apparatus provided in an embodiment of this application. As shown in FIG. 12, a body fat scale controlling apparatus 500 can perform the body fat scale controlling method shown in FIG. 10 and FIG. 11. The body fat scale controlling apparatus 500 includes an obtaining module 510, an analysis module 520, and a recognition module 530. The obtaining module 510 is configured to: when the number of uses of the body fat scale reaches a preset number and it is detected that a user is in a stable state on the body fat scale, obtain the first initial data of the user, where the first initial data includes bioimpedance data and pressure distribution data.

The analysis module 520 is configured to input the first initial data, historical use data, and user preference analysis data into a preset recognition model to determine a target fingerprint recognizer suitable for the user.

The recognition module 530 is configured to: in response to a fingerprint enrollment operation of the user via the target fingerprint recognizer, obtain the first fingerprint image of the user and determine the first identifier corresponding to the first fingerprint image, where the target fingerprint recognizer is the first fingerprint recognizer or the second fingerprint recognizer. The recognition module 530 is further configured to: based on the first identifier, establish a matching relationship between the first initial data and basic data corresponding to the first identifier, to determine the indicator data of the user.

Each module of the body fat scale controlling apparatus 500 can respectively perform corresponding steps in the foregoing method embodiments. Therefore, details of the modules are not described herein again. For details, refer to descriptions of the foregoing corresponding steps.

It should be noted that the foregoing body fat scale controlling apparatus 500 is embodied in a form of a functional module. The term "module" herein may be implemented in the form of software and/or hardware, and this is not specifically limited.

For example, the "module" may be a software program, a hardware circuit, or a combination thereof that implements the foregoing functions. A hardware circuit may include an application-specific integrated circuit (application-specific integrated circuit, ASIC), an electronic circuit, a processor (for example, a shared processor, a dedicated processor, or a group of processors) for executing one or more software or firmware programs, a memory, a combined logic circuit, and/or another suitable component that supports the described function.

FIG. 13 is a schematic structural diagram of a body fat scale provided in this application. The dashed lines in FIG. 13 indicate that a unit or a module is optional. The body fat scale 600 can be configured to implement the body fat scale controlling method described in the foregoing method embodiments.

The body fat scale 600 includes one or more processors 601, and the one or more processors 601 can support the body fat scale 600 in implementing the body fat scale controlling method in the method embodiments. The processor 601 may be a general-purpose processor or a dedicated processor. For example, the processor 601 may be a central processing unit (central processing unit, CPU), a digital signal processor (digital signal processor, DSP), an application specific integrated circuit (application specific integrated circuit, ASIC), a field-programmable gate array (field-programmable gate array, FPGA), or another programmable logic device.

The processor 601 can be configured to control the body fat scale 600, execute a software program, and process data of the software program. The body fat scale 600 may further include a communication module 605 for implementing input (receiving) and output (sending) of signals.

The body fat scale 600 may include one or more memories 602, storing a program 604. The program 604 can be run by the processor 601 to generate an instruction 603, so that the processor 601 performs the body fat scale controlling method described in the foregoing method embodiments according to the instruction 603.

This application further provides a computer program product, where when the computer program product is executed by the processor 601, the body fat scale controlling method according to any one of the method embodiments in this application is implemented. The computer program product may be stored in the memory 602, and is, for example, the program 604. The program 604 is finally converted, via processing procedures such as preprocessing, compilation, assembly, and linking, into a target file that can be executed by the processor 601.

This application further provides a computer-readable storage medium, storing a computer program. When the computer program is executed by a computer, the body fat scale controlling method according to any one of the method embodiments in this application is implemented. The computer program may be a senior language program or an executable target program. The computer-readable storage medium is, for example, the memory 602.

In several embodiments provided in this application, it should be understood that the disclosed apparatus and method may be implemented in other manners. For example, the apparatus embodiments described above are merely illustrative. For example, the division of units is only a logical function division, and there may be another division method in actual implementation. In addition, the shown or discussed mutual couplings or direct couplings or communication connections may be implemented by using some interfaces. The indirect couplings or communication connections between the apparatuses or units may be implemented in electronic, mechanical, or other forms.

The foregoing descriptions are only specific embodiments of this application, but are not intended to limit the protection scope of this application. Any change or replacement readily figured out by persons skilled in the art within the technical scope disclosed in this application shall fall within the protection scope of this application. Therefore, the protection scope of this application shall be subject to the protection scope of the claims.

## Claims

1. A body fat scale, comprising a biometric recognizer, a measurement device, a controller, and a communication module, wherein the controller is communicatively connected to the biometric recognizer, the measurement device, and the communication module;
the biometric recognizer comprises a first fingerprint recognizer and a second fingerprint recognizer, wherein the first fingerprint recognizer is disposed on the first surface of the body fat scale, and the first fingerprint recognizer comprises a first fingerprint recognition panel adjustable by an angle and a first sensor; and the second fingerprint recognizer is disposed on a side surface of the body fat scale, the second fingerprint recognizer comprises a second fingerprint recognition panel and a second sensor, and there is a first inclination angle between the second fingerprint recognition panel and a vertical plane;
the measurement device comprises an electrode and a pressure sensor; and
the controller is configured to:
when the number of uses of the body fat scale reaches a preset number and it is detected that a user is in a stable state on the body fat scale, obtain first initial data of the user, wherein the first initial data comprises bioimpedance data and pressure distribution data;
input the first initial data, historical use data, and user preference analysis data into a preset recognition model to determine a target fingerprint recognizer suitable for the user;
in response to a fingerprint enrollment operation of the user via the target fingerprint recognizer, obtain the first fingerprint image of the user and determine the first identifier corresponding to the first fingerprint image, wherein the target fingerprint recognizer is the first fingerprint recognizer or the second fingerprint recognizer; and
based on the first identifier, establish a matching relationship between the first initial data and basic data corresponding to the first identifier, and determine indicator data of the user.

2. The body fat scale according to claim 1, wherein the first fingerprint recognizer further comprises a universal connection mechanism, the universal connection mechanism is configured to movably connect the first fingerprint recognition panel to the body fat scale, the first fingerprint recognition panel is provided with a first sensor, and the second fingerprint recognition panel is provided with the second sensor.

3. The body fat scale according to claim 2, wherein the universal connection mechanism further comprises an angle sensor and a motor, and after determining the target fingerprint recognizer suitable for the user, the controller is further configured to:
if the target fingerprint recognizer is the first fingerprint recognizer, analyze historical use data to determine a target angle of the first fingerprint recognition panel, wherein the historical use data comprises use frequency of the first fingerprint recognizer and an initial angle that is obtained via the angle sensor when the user enrolls a fingerprint and that corresponds to the first fingerprint recognition panel; and
control the motor to adjust the angle of the first fingerprint recognition panel to the target angle.

4. The body fat scale according to claim 2, wherein after obtaining the first fingerprint image of the user in response to the fingerprint enrollment operation of the user via the target fingerprint recognizer, the controller is further configured to:
identify and analyze the first fingerprint image based on user information stored in the body fat scale;
if the first fingerprint image is not recognized, perform a matching degree analysis respectively on a characteristic of the first fingerprint image and characteristics of fingerprints that have been collected by the first fingerprint recognizer and the second fingerprint recognizer, to obtain a corresponding similarity; and
use a fingerprint recognizer with the highest similarity as an updated target fingerprint recognizer.

5. The body fat scale according to claim 1, wherein when the number of uses of the body fat scale does not reach a preset number and it is detected that the user is in a stable state on the body fat scale, the controller is further configured to:
obtain the second fingerprint image and the second initial data of the user, wherein the second fingerprint image is an image collected via the first fingerprint recognizer and/or the second fingerprint recognizer;
analyze the second fingerprint image and the second initial data to obtain the historical use data and user preference analysis data of the user; and
optimize the preset recognition model based on the second fingerprint image, the second initial data, the historical use data, and the user preference analysis data, to obtain an optimized preset recognition model.

6. The body fat scale according to claim 1, wherein the first fingerprint recognizer further comprises a first indicator light, and the second fingerprint recognizer further comprises a second indicator light; and after determining the target fingerprint recognizer suitable for the user, the controller is further configured to:
generate prompt information, wherein the prompt information is used to instruct the user to enroll a fingerprint via the target fingerprint recognizer; and
when the prompt information indicates that the first fingerprint recognizer is the target fingerprint recognizer, drive the first indicator light to emit light based on a preset mode; or
when the prompt information indicates that the second fingerprint recognizer is the target fingerprint recognizer, drive the second indicator light to emit light based on the preset mode, wherein
the preset mode comprises a constant-on mode or a flashing mode.

7. The body fat scale according to any one of claims 1 to 6, wherein the first fingerprint recognizer further comprises a first LED light source, and the first sensor comprises an optical fingerprint sensor and a capacitive fingerprint sensor; and
the controller is further configured to:
obtain heart rate data of the user, wherein the heart rate data is used to obtain the historical use data of the user to optimize the preset recognition model, and to be input into the preset recognition model for determining the target fingerprint recognizer suitable for the user.

8. The body fat scale according to any one of claims 1 to 6, wherein the body fat scale comprises a handle and a scale body; and
the biometric recognizer further comprises a facial recognizer, the first fingerprint recognizer and the facial recognizer are disposed on the second surface of the handle, and the second fingerprint recognizer is disposed on the side surface of the scale body.

9. The body fat scale according to any one of claims 1 to 6, wherein the first sensor comprises an optical fingerprint sensor and a capacitive fingerprint sensor, and the second sensor comprises an optical fingerprint sensor and a capacitive fingerprint sensor.

10. The body fat scale according to any one of claims 1 to 6, wherein the biometric recognizer further comprises a toe print recognizer, the toe print recognizer is disposed on the first surface of the body fat scale, and the user performs a toe print enrollment operation via the toe print recognizer, to obtain a toe print image of the user and determine the first identifier corresponding to the toe print image.

11. The body fat scale according to claim 10, wherein the toe print recognizer comprises the third fingerprint recognition panel adjustable by an angle and the third sensor, the third fingerprint recognition panel is movably connected to the body fat scale via a universal connection mechanism, and the third fingerprint recognition panel is provided with the third sensor.

12. The body fat scale according to claim 11, wherein the third sensor comprises an optical fingerprint sensor and a capacitive fingerprint sensor.

13. A body fat scale controlling method, applied to the body fat scale according to any one of claims 1 to 12, wherein the body fat scale controlling method comprises:
obtaining the first initial data of the user when the number of uses of the body fat scale reaches a preset number and it is detected that a user is in a stable state on the body fat scale, wherein the first initial data comprises bioimpedance data and pressure distribution data;
inputting, by the body fat scale, the first initial data, historical use data, and user preference analysis data into a preset recognition model to determine a target fingerprint recognizer suitable for the user;
in response to a fingerprint enrollment operation of the user via the target fingerprint recognizer, obtaining, by the body fat scale, the first fingerprint image of the user and determining the first identifier corresponding to the first fingerprint image, wherein the target fingerprint recognizer is the first fingerprint recognizer or the second fingerprint recognizer; and
based on the first identifier, establishing, by the body fat scale, a matching relationship between the first initial data and basic data corresponding to the first identifier, and determining indicator data of the user.
